# EUROPEAN PATENT APPLICATION

(11) **EP 1 964 549 A1**
(43) Date of publication of application: **03.09.2008**
(21) Application number: 06834997.6
(22) Date of filing: 20.12.2006
(51) Int. Cl.: A61K 9/26, A61K 31/07, A61K 31/121, A61K 31/122, A61K 31/355, A61K 31/59, A61K 47/04, A61K 47/10, A61K 47/26, A61K 47/38

(54) **ORALLY DISINTEGRATING TABLET COMPRISING FAT-SOLUBLE SUBSTANCE**

(30) Priority: 20.12.2005 JP 2005366791
(71) Applicant: Eisai R&D Management Co., Ltd., Tokyo 112-8088 (JP)
(72) Inventor: TAKAMI, Norishige, Kakamigahara-shi, Gifu 5016195 (JP); NAGIRA, Shinsuke, Kakamigahara-shi, Gifu 5016195 (JP); OKADA, Yasushi, Kakamigahara-shi, Gifu 5016195 (JP); OHWAKI, Takayuki, Kakamigahara-shi, Gifu 5016195 (JP); NISHIJIMA, Reiko, Kakamigahara-shi, Gifu 501-6195 (JP)
(74) Representative: HOFFMANN EITLE
(86) International application number: PCT/JP2006/325327
(87) International publication number: WO 2007/072840

(57) **Abstract**

The present invention provides an orally rapid disintegrating tablet preparation that contains a high dose of a fat-soluble active ingredient, that exhibits excellent disintegration characteristics in the oral cavity, and that can be produced by a dry tabletting method. The present invention also provides a method of producing an orally rapid disintegrating tablet preparation. The present invention discloses an orally rapid disintegrating tablet preparation that is obtained by tabletting a uniform mixture prepared by mixing saccharide alcohol, crystalline cellulose, and a lubricant with a granule that has been produced by the adsorption of a fat-soluble active ingredient on a porous material.

## Description

### Technical Field

The present invention relates to an orally rapid disintegrating tablet preparation that contains a fat-soluble active ingredient, particularly an oily active ingredient. More particularly, the present invention relates to an orally rapid disintegrating tablet preparation that blends a high content of the fat-soluble active ingredient.

### Background Art

An orally rapid disintegrating tablet preparation is a tablet that disintegrates rapid under an action of saliva in the oral cavity and is a dosage form that is well adapted for administration to the elderly and infants with their weak swallowing force. Moreover, since water is not required for its ingestion, the orally rapid disintegrating tablet preparation offers the advantage of enabling ingestion at any time and any place.

Various investigations have already been carried out on the orally rapid disintegrating tablet preparations and quite a few reports have already appeared on this subject. For example, the orally rapid disintegrating tablet preparation has been known that can be produced by introducing an active ingredient suspended in an aqueous hydrolyzed gelatin solution into a cylindrical aluminum mold that has been cooled by liquid nitrogen and reducing the pressure after freezing (refer, for example, to Patent Document 1). The rapid disintegrating tablet has been also known that can be produced by introducing mannitol, lactose, and an active ingredient suspended in aqueous agar solution into a mold cavity and reducing the pressure and removing the water fraction (refer, for example, to Patent Document 2). However, there are drawbacks to these methods, such as requirements for special facilities, high costs, and inability to obtain a satisfactory tablet hardness.

The orally dissolving tablet has also been disclosed that can be produced by tabletting a mixture of an active ingredient, a saccharide, and water in an amount that wets a surface of the saccharide (refer, for example, to Patent Document 3). When this method is used, the moist powder containing the active ingredient sticks to the punch and die elements of the tabletting machine and there are substantial variations in the tablet weight. In addition, the active ingredient is prone to bleed to the tablet surface when a high content of fat-soluble active ingredient is incorporated and particularly when a high content of an oily active ingredient is incorporated.

Patent Document 4 describes a tablet prepared by emulsifying a fat-soluble substance with a water-soluble polymer substance and adding an adsorbent to prepare a suspension; particulating this suspension by spray drying; adding diluent, disintegrant, binder, and the like; and tabletting. Patent Document 5 describes a tablet prepared by adsorbing teprenone to silicic acids; adding thereto diluent, disintegrant, binder, and the like; granulating with water or organic solvent; drying and executing size adjustment; adding diluent, disintegrant, lubricant, and the like; and tabletting.
The use of these methods can effectively inhibit bleed out of the fat-soluble active ingredient when the above powder is tabletted, but these methods give tablets with long tablet disintegration times and cannot be used to produce orally disintegrating tablets.

The tablets are disclosed in Patent Document 6 that can be obtained by granulating a saccharide alcohol or saccharide having an average particle diameter no greater than 30 µm, an active ingredient, and a disintegrant; adding magnesium stearate; and compressing. The tablet are also disclosed in Patent Document 6 that can be obtained by mixing a saccharide alcohol or saccharide having an average particle diameter no greater than 30 µm, an active ingredient, a disintegrant, and a disintegration auxiliary; compressing; and volatilizing the disintegration auxiliary.
The rapid disintegrating solid preparations comprising an active ingredient, a saccharide or saccharide alcohol having an average particle diameter of 30 to 300 µm, a disintegrant, and cellulose are described in Patent Document 7. The orally rapid disintegrating tablets are described in Patent Document 8 that contain crystalline cellulose and saccharide alcohol in the range of 5:5 to 3.5:6.5 and that does not contain a disintegrant. However, when the fat-soluble active ingredient is incorporated as an active ingredient in these tablets, the active ingredient bleeds out during tabletting and sticks to the punch and die elements of the tabletting machine, and/or a satisfactory hardness is not obtained, and/or a long disintegration time is obtained, and as a consequence these tablets are impractical as orally rapid disintegrating tablets.

Patent Document 1: Japanese Patent Application Laid-open No. S 53-44619
Patent Document 2: WO 93/12769
Patent Document 3: Japanese Patent Application Laid-open No. H5-271054
Patent Document 4: Japanese Patent Application Laid-open No. 2003-313145
Patent Document 5: Japanese Patent Application Laid-open No. 2000-16934
Patent Document 6: WO 97/47287
Patent Document 7: Japanese Patent Application Laid-open No. 2001-58944
Patent Document 8: Japanese Patent Application Laid-open No. H11-199517

### Disclosure of Invention

### Problems to be Solved by the Invention

As described above, while there are a number of reports on the orally rapid disintegrating tablets, the incorporation of the fat-soluble active ingredient can still cause problems such as inability to obtain a satisfactory hardness even with tabletting, bleed out of the fat-soluble active ingredient to the surface of the tablet during storage, a lengthening of the disintegration time, and instability in the active ingredient leading to its degradation. Accordingly, a practical orally rapid disintegrating tablet having a high fat-soluble active ingredient dose remains unrealized at the present time.

### Means for Solving the Problems

In view of these circumstances, the present inventors carried out intensive investigations into the orally rapid disintegrating tablets containing the fat-soluble active ingredient and as a result discovered that the problems cited above can be solved by the structure given below, thereby leading to the present invention achieved based on this discovery.

That is, the present invention relates to an orally rapid disintegrating tablet preparation obtained by tabletting a mixture comprising a saccharide, crystalline cellulose, a lubricant, and a granule of a fat-soluble active ingredient adsorbed to an adsorbent.
The following aspects are also encompassed by the present invention.
(1) The above orally rapid disintegrating tablet preparation, wherein the fat-soluble active ingredient is at least one selected from the group consisting of vitamin A, vitamin D, vitamin E, vitamin K, teprenone, and coenzyme Q.
(2) The above orally rapid disintegrating tablet preparation, wherein the fat-soluble active ingredient is an oily active ingredient.
(3) The above orally rapid disintegrating tablet preparation, wherein the oily active ingredient is vitamin A, vitamin E, or teprenone.
(4) The above orally rapid disintegrating tablet preparation, wherein the fat-soluble active ingredient is contained at from 5 to 75 % by weight based on a total weight of the tablet preparation.
(5) The above orally rapid disintegrating tablet preparation, wherein the adsorbent is at least one selected from the group consisting of calcium silicate, hydrated silicon dioxide, and light anhydrous silicic acid.
(6) The above orally rapid disintegrating tablet preparation, wherein the saccharide is a saccharide alcohol.
(7) The above orally rapid disintegrating tablet preparation, wherein the saccharide alcohol is erythritol or mannitol.
(8) The above orally rapid disintegrating tablet preparation, wherein the saccharide alcohol is mannitol.
(9) The above orally rapid disintegrating tablet preparation, wherein an average particle diameter of the saccharide alcohol is from 300 µm to 700 µm.
(10) The above orally rapid disintegrating tablet preparation, wherein a content of the saccharide alcohol is from 5 to 50 % by weight based on of a total weight of the tablet preparation.
(11) The above orally rapid disintegrating tablet preparation, wherein the crystalline cellulose is contained at from 1 to 20 weight parts based on 1 weight part of the saccharide alcohol.
(12) The above orally rapid disintegrating tablet preparation, wherein the adsorbent is contained at from 0.1 to 10 weight parts based on 1 weight part of the fat-soluble active ingredient.
(13) The above orally rapid disintegrating tablet preparation, wherein the adsorbent is contained at from 0.5 to 2 weight parts based on 1 weight part of the fat-soluble active ingredient.
(14) The above orally rapid disintegrating tablet preparation, wherein a hardness of the table preparation is at least 30 N and an intraoral disintegration time in the disintegration test procedure described in the Japanese Pharmacopoeia, Fourteenth Edition, is not more than 30 seconds.
(15) A method of stabilizing an orally rapid disintegrating tablet preparation, comprising the steps of:
   adding a saccharide, crystalline cellulose, and a lubricant to and mixing same with a granule comprising a fat-soluble active ingredient adsorbed on an adsorbent; and
   tabletting the mixture.
(16) A process of producing an orally rapid disintegrating tablet preparation, comprising the steps of:
   adding a saccharide, crystalline cellulose, and a lubricant to and mixing same with a granule comprising a fat-soluble active ingredient adsorbed on an adsorbent; and
   tabletting the mixture.

The term "orally rapid disintegrating tablet preparation" used in the present invention refers to a preparation having an intraoral disintegration or dissolution time (time required for the tablet to undergo complete disintegration by an action of the saliva in the oral cavity of healthy adult men or women) of generally from about 5 to 120 seconds, preferably from about 5 to 60 seconds, and more preferably from about 5 to 40 seconds, although this time depends on the size and thickness of the tablet itself.

### Advantageous Effects of the Invention

The tablet preparation obtained according to the present invention by making a granule in which a fat-soluble active ingredient is adsorbed on an adsorbent and tabletting a mixture that combines this granule with a saccharide, crystalline cellulose, and a lubricant, provides an orally rapid disintegrating tablet preparation that exhibits a short disintegration time and a satisfactory strength and that is free of bleed out of the active ingredient. In particular, the orally rapid disintegrating tablet preparation according to the present invention can stably contain the fat-soluble active ingredient at high concentrations of from 5 to 75 % by weight based on the total weight of the tablet preparation. Moreover, due to its ease of disintegration in the oral cavity, the tablet preparation according to the present invention can be easily ingested even by infants and the elderly, who have a low swallowing force. The tablet preparation according to the present invention also prevents the fat-soluble active ingredient from bleeding out during production and storage. In addition, wear damage of the tablet preparation during storage and transport can be prevented because an acceptable tablet hardness can be obtained. The orally rapid disintegrating tablet preparation according to the present invention is thus able to provide an excellent orally rapid disintegrating tablet. Furthermore, the orally rapid disintegrating tablet preparation according to the present invention can be produced using the conventional tablet machines and does not require special equipments.

### Best Mode for Carrying Out the Invention

Embodiments of the present invention will be described, but the following embodiments are illustrative for the purpose of describing the present invention and the present invention should not be limited only to these embodiments. The present invention can be implemented by a variety of modes while not departing from the spirit and gist of the present invention.

Fat-soluble active ingredients in the present invention may be a solid, liquid, or oil at ambient temperature.
The fat-soluble active ingredients are not particularly limited and can be exemplified by the carotene group and vitamin A group, e.g., retinol, retinoic acid, and the like; the vitamin D group, e.g., cholecalciferol, ergocalciferol, and the like; the vitamin E group, e.g., tocopherol, tocotrienol, and the like; the vitamin K group, e.g., phytonadione, menaquinone, menadione, and the like; coenzyme Qs, e.g., ubiquinone and the like; terpenes, e.g., teprenone and the like; as well as eicosapentaenoic acid, docosahexaenoic acid, nifedipine, gefarnate, plaunotol, clofibrate, nitroglycerin, flurbiprofen, ibuprofen, amiodarone, simvastatin, indomethacin farnesyl, cod liver oil, and the like. Vitamin A, vitamin D, vitamin E. vitamin K, teprenone, coenzyme Q, and indomethacin farnesyl are preferred, and vitamin A, vitamin E, teprenone, and indomethacin farnesyl, which are oils at ambient temperature, are particularly preferred.
The content of the fat-soluble active ingredient based on a total weight of tablet may be from 5 to 75 % by weight and is preferably from 5 to 50 % by weight and more preferably from 5 to 30 % by weight.

The adsorbent may be used in the present invention for the purpose of adsorbing and retaining the fat-soluble active ingredient that is a main drug. Examples of the adsorbent include silicic acids such as calcium silicate, hydrated silicon dioxide, light anhydrous silicic acid, magnesium silicate, magnesium metasilicate aluminate, and the like. Calcium silicate, hydrated silicon dioxide, and light anhydrous silicic acid are preferred.
Calcium silicate is commercially available as Florite R (Tokuyama Corp.); hydrated silicon dioxide is commercially available as Sylysia (Fuji Silysia Chemical Co., Ltd.) and Carplex (Shionogi & Co., Ltd); and light anhydrous silicic acid is commercially available as Aerosil (Nippon Aerosil); and these can therefore be readily acquired.
The adsorbent is used at from 0.1 to 10 parts by weight and preferably from 0.5 to 2 parts by weight based on 1 part by weight of the fat-soluble active ingredient.

Saccharide used in the present invention refers to saccharides and saccharide alcohols. Examples of the saccharide include sucrose, fructose, glucose, trehalose, oligosaccharides, raffinose, and the like. Examples of the saccharide alcohol include xylitol, erythritol, lactitol, mannitol, maltitol, sorbitol, reduced palatinose, and the like. The saccharide alcohol is preferred and erythritol and mannitol are particularly preferred.

The average particle diameter of the saccharide or saccharide alcohol is generally from 50 to 1500 µm, preferably from 50 to 800 µm and more preferably from 300 to 700 µm. Considering, for example, the use of mannitol as the saccharide alcohol, mannitol can be readily available, for example, the commercially available Mannit S (Towa Kasei, average particle diameter = 170 µm), Pearlitol DC400 (Roquette Japan, average particle diameter = 400 µm), and Pearlitol DC500 (Roquette Japan, average particle diameter = 500 micrometer).
The amount of the saccharide or saccharide alcohol based on the total weight of the tablet is from 5 to 50 % by weight and preferably from 20 to 50 % by weight.

Crystalline cellulose used in the present invention refers to the material obtained by the depolymerization and purification of the α-cellulose obtained from a plant as the starting material and can be readily available, for example, the commercially available Avicel PH101, Avicel PH102, Ceolus (Asahi Kasei Chemicals Corporation), and FarmaCell 101 (Gokyo Trading Co., Ltd.).
The amount of crystalline cellulose based on the total weight of the tablet is from 20 to 70 % by weight and preferably from 30 to 60 % by weight.

Regarding the blend ratio between the saccharide alcohol and the crystalline cellulose in the present invention, the amount of crystalline cellulose is from 1 to 20 parts by weight based on 1 part by weight of the saccharide alcohol. When the blend amount of the saccharide alcohol exceeds that of the crystalline cellulose, a satisfactory hardness is not obtained upon tabletting, or, if the hardness is acceptable, a long disintegration time is exhibited.

The lubricant used in the present invention refers to a lubricant as commonly used in the pharmaceutical field. Examples of the lubricant include magnesium stearate, calcium stearate, sodium stearyl fumarate, talc, synthetic magnesium silicate, carnauba wax, hydrogenated oils, microcrystalline wax, and the like.
The blend amount of lubricant based on the total weight of the tablet is from 0.05 to 1 % by weight and preferably from 0.1 to 0.5 % by weight.

In addition to the above, the orally rapid disintegrating tablet preparation according to the present invention can blend, insofar as the object of the present invention is not impaired, the excipients commonly used in tablet production, for example, binders, disintegrants, taste enhancers, colorants, oxidation inhibitors, and the like.

Examples of the binder may include hydroxypropyl cellulose, polyvinyl alcohol, povidone, copolyvidone, gum arabic, guar gum, corn starch, and the like.

Examples of the disintegrant may include crosspovidone, crosscarmellose sodium, crystalline cellulose, low-substituted hydroxypropyl cellulose, and the like.

Examples of the taste enhancer may include aspartame, stevia, cacao powder, licorice, dipotassium glycyrrhizinate, menthol, borneol, and the like.

Examples of the colorant may include ferric oxide yellow, ferric oxide red, red iron oxide, carmine, sodium riboflavin phosphate, cochineal, carotene, Food Blue No. 1, and the like.

Examples of the oxidation inhibitor may include ascorbic acid, erythorbic acid, tocopherols, propyl gallate, and the like.

The process of producing the orally rapid disintegrating tablet preparation according to the present invention is described in the following.
First, the fat-soluble active ingredient is emulsified with the addition of an aqueous solution that optionally contains a binder, e.g., polyvinylpyrrolidone, polyvinyl alcohol, and the like, and this emulsion is added to the adsorbent, e.g., a silicic acid, in order to adsorb the fat-soluble active ingredient. Purified water is added thereto and granulation is carried out to produce granules. At this time, the surface of the granules may be coated with a suitably selected coating agent. In addition, the silicic acid may be granulated and thereafter the fat-soluble active ingredient may be sprayed thereon.
The saccharide, e.g., mannitol, erythritol, and the like, crystalline cellulose, and the lubricant are added to the adsorbed granules described above and, after thorough mixing, tabletting is performed. Excipient, e.g., binder, disintegrant, sweetener, taste enhancer, colorant, and the like, may be added as appropriate at this time.

The equipment ordinarily used for tablet molding or agglomeration can be used for tabletting in the process of producing the orally rapid disintegrating tablet preparation according to the present invention. More specifically, a single-stroke tabletting machine or a rotary tabletting machine can be used. The compression force during tabletting is generally from 50 to 10,000 kg, preferably from 100 to 5,000 kg, and more preferably from 200 to 3,000 kg.

The hardness (value measured with a tablet hardness meter; unit: N) of the orally rapid disintegrating tablet preparation according to the present invention is generally from 10 to 100 N, preferably from 20 to 100 N, and more preferably from 30 to 100 N. The disintegration time (value measured using the disintegration test procedure described in the Japanese Pharmacopoeia, Fourteenth Edition) of the orally rapid disintegrating tablet preparation according to the present invention is from 5 to 120 seconds, preferably from 5 to 60 seconds, and more preferably from 5 to 30 seconds.

### EXAMPLE

The present invention is described in detail through the examples that follow, but the present invention is not limited to these examples.

### Example 1

72 g of polyvinyl alcohol (GOHSENOL, Nippon Synthetic Chemical Industry Co., Ltd.) was dissolved in 720 g of purified water; 300 g of teprenone and 1.5 g of tocopherol were added thereto; and emulsification was carried out using a multipurpose homogenizer (Polytron, Kobayashi Trading). The resulting emulsion and 300 g of hydrated silicon dioxide (Sylysia 350, Fuji Silysia Chemical Co., Ltd.) were placed into a stirred-type mixer (10-L Super Mixer, Kawata Mfg. Co., Ltd.) and granulation was carried out. The granules were dried with a fluid bed dryer (Flow Coater FLO-5, Freund Corporation) followed by size adjustment with a Power Mill (Showa Giken Ltd.) to give adsorbed granules.
340 g of these adsorbed granules was placed into a stirred-type mixer (10-L Super Mixer, Kawata Mfg. Co., Ltd.) and 220 g of a 9% aqueous methyl cellulose solution, purified water, and 25 g of hydrated silicon dioxide were added thereto and the granules were coated.
To 112 g of the coated granules were added 0.7 g of menthol flavorant (Menthol Coaton AL21717, Ogawa & Co., Ltd.), 2 g of magnesium stearate (Taihei Chemical Industrial Co., Ltd.), 320 g of crystalline cellulose (Avicel PH101, Asahi Kasei Chemicals Corporation), 210 g of mannitol (Mannit S, Towa Kasei), and 50 g of corn starch and, after thorough mixing, tablets (695 mg per tablet) were prepared by tabletting at a compression force of 1000 kg using a rotary tabletting machine (AP-15, Hata Ironworks Co., Ltd.).

### Example 2

500 g of calcium silicate (Florite R, Tokuyama Corp.) was placed into a stirred-type mixer (25-L vertical mixer, Powrex Corp.); 1010 g of teprenone and 2 g of tocopherol were added thereto and adsorption was carried out; and purified water was then added and granulation was carried out. This was dried with a fluid bed dryer (Flow Coater FLO-5, Freund Corporation) followed by size adjustment with a Power Mill to give adsorbed granules.
150 g of these adsorbed granules, 336 g of mannitol (Pearlitol DC400, Roquette Japan), 512 g of crystalline cellulose (Avicel PH101, Asahi Kasei Chemicals Corporation), 80 g of corn starch, 50 g of calcium silicate, and 3.2 g of sodium stearyl fumarate (Kimura Sangyo Co., Ltd.) were mixed and tablets (566 mg per tablet) were prepared by tabletting at a compression force of 650 kg using a rotary tabletting machine.

### Example 3

1000 g of calcium silicate was placed into a stirred-type mixer; 1000 g of teprenone and 2 g of tocopherol were added thereto and adsorption was carried out; and purified water was then added and granulation was carried out. This was dried with a fluid bed dryer (Flow Coater FLO-5, Freund Corporation) followed by size adjustment with a Power Mill to give adsorbed granules.
300 g of these adsorbed granules, 504 g of mannitol (Pearlitol DC500, Roquette Japan), 768 g of crystalline cellulose (Avicel PH102, Asahi Kasei Chemicals Corporation), 120 g of corn starch, and 4.8 g of sodium stearyl fumarate were mixed and tablets (566 mg per tablet) were prepared by tabletting at a compression force of 550 kg using a rotary tabletting machine.

### Example 4

500 g of calcium silicate was placed into a stirred-type mixer; 1000 g of teprenone and 2 g of tocopherol were added thereto and adsorption was carried out; and a solution of 200 g of hydroxypropyl cellulose (HPC-L, Nippon Soda Co., Ltd.) dissolved in purified water was then added and granulation was carried out. This was dried with a fluid bed dryer (Flow Coater FLO-5, Freund Corporation) followed by size adjustment with a Power Mill to give adsorbed granules.
255 g of these adsorbed granules, 504 g of mannitol (Pearlitol DC500), 768 g of crystalline cellulose (Avicel PH102), 75 g of calcium silicate, 120 g of corn starch, and 4.8 g of sodium stearyl fumarate were mixed and tablets (576 mg per tablet) were prepared by tabletting at a compression force of 380 kg using a rotary tabletting machine.

### Example 5

700 g of calcium silicate was placed into a stirred-type mixer; 700 g of tocopherol was added thereto and adsorption was carried out; and a solution of 140 g of hydroxypropyl cellulose dissolved in an appropriate amount of purified water was then added and granulation was carried out. This was dried with a fluid bed dryer (Flow Coater FLO-5, Freund Corporation) followed by size adjustment with a Power Mill to give adsorbed granules.
440 g of these adsorbed granules, 600 g of mannitol (Pearlitol DC500), 920 g of crystalline cellulose (Avicel PH102), 160 g of corn starch, 100 g of low-substituted hydroxypropyl cellulose, 100 g of hydrated silicon dioxide, 60 g of aspartame, and 2.4 g of flavorant were mixed. For each 100 g of the resulting mixture, 1.5 g of magnesium stearate was added with mixing, and the mixture thus obtained was subjected to tabletting at a compression force of 600 kg using an Autograph (AG-5000, Shimadzu) to give tablets (600 mg per tablet).

### Example 6

400 g of hydrated silicon dioxide was placed into a stirred-type mixer; 400 g of menatetrenone was added thereto and adsorption was carried out; and a solution of 80 g of hydroxypropyl cellulose dissolved in an appropriate amount of purified water was then added and granulation was carried out. This was dried with a rack-type dryer followed by size adjustment with a Power Mill to give adsorbed granules.
440 g of these adsorbed granules, 600 g of mannitol (Pearlitol DC500), 920 g of crystalline cellulose (Avicel PH102), 160 g of corn starch, 100 g of low-substituted hydroxypropyl cellulose, 100 g of hydrated silicon dioxide, 60 g of aspartame, 2.4 g of flavorant, and 8 g of magnesium stearate were mixed, and the mixture thus obtained was subjected to tabletting at a compression force of 600 kg using an Autograph (AG-5000, Shimadzu) to give tablets (600 mg per tablet).

### Comparative Example 1

50 g of hydrated silicon dioxide was placed into a stirred-type mixer (10-L Super Mixer) and 50 g of teprenone and 0.1 g of tocopherol were added thereto and adsorption was carried out. 360 g of crystalline cellulose (Avicel PH101) and 237 g of erythritol (Nikken Chemical Laboratory) were then added with mixing. Purified water was then added and granulation was carried out. This was dried with a fluid bed dryer (Flow Coater FLO-5, Freund Corporation) followed by size adjustment with a Power Mill to give adsorbed granules.
To 697.1 g of these adsorbed granules were added 0.4 g of stevia (Maruzen Pharmaceuticals Co., Ltd.), 0.7 g of Menthol Coaton (Ogawa & Co., Ltd.), and 2.0 g of magnesium stearate (Taihei Chemical Industrial Co., Ltd.); mixing was followed by tabletting at a compression force of 330 kg using a rotary tabletting machine (AP-13, Hata Ironworks Co., Ltd.) to give tablets (700 mg per tablet).

### Comparative Example 2

100 g of hydrated silicon dioxide (Sylysia 350) was placed into a stirred-type mixer (20-L Super Mixer) and 100 g of teprenone and 0.2 g of tocopherol were added thereto and adsorption was carried out. 660 g of crystalline cellulose (Avicel PH102) and 534.8 g of mannitol (Mannit P, Towa Kasei) were then added with mixing. Purified water was then added and granulation was carried out. This was dried with a Flow Coater followed by size adjustment with a Power Mill to give adsorbed granules.
To 1046.25 g of these adsorbed granules were added 1.05 g of Menthol Coaton and 3 g of magnesium stearate; mixing was followed by tabletting at a compression force of 800 kg using a rotary tabletting machine (AP-13) to give tablets (700 mg per tablet).

### Comparative Example 3

72 g of polyvinyl alcohol was dissolved in 720 g purified water followed by the addition of 300 g of teprenone and 1.5 g of tocopherol and emulsification (Polytron, Kobayashi Trading). The resulting emulsion and 300 g of hydrated silicon dioxide (Sylysia 350, Fuji Silysia Chemical Co., Ltd.) were placed into a stirred-type mixer (10-L Super Mixer, Kawata Mfg. Co., Ltd.) and granulation was carried out. The granulate was dried with a Flow Coater followed by size adjustment with a Power Mill to give adsorbed granules.
340 g of these adsorbed granules was placed into a stirred-type mixer (10-L Super Mixer, Kawata Mfg. Co., Ltd.) and 220 g of a 9% aqueous methyl cellulose solution, purified water, and 25 g of hydrated silicon dioxide were added and the granules were coated.
To 325 g of the coated granules was added 790 g of mannitol (Mannit P), and the material obtained by kneading with an 18% povidone-ethanol solution was used to produce tablets by filling into mold cavities (diameter = 9.5 mm, thickness = 4.0 mm) using a wet tabletting machine (refer to Japanese Patent Application Laid-open No. H 8-19588) and applying a pressure of 35 kg. Drying then gave tablets (300 mg per tablet).

### Comparative Example 4

72 g of polyvinyl alcohol was dissolved in 500 g of purified water followed by the addition of 300 g of teprenone and 0.6 g of tocopherol and emulsification. 300 g of hydrated silicon dioxide was placed into a stirred-type mixer (10-L Super Mixer) and the emulsion and a suitable amount of purified water were then added thereto and stirring and granulation were carried out. Drying and size adjustment then gave granules. Adsorption was carried out by the addition of 264 g of a 9% aqueous solution of polyvinyl alcohol and 24 g of Sylysia to 340 g of the granules; drying then gave adsorbed granules.
400 g of erythritol, 600 g of crystalline cellulose (Avicel PH101), and 100 g of corn starch were mixed using a stirred-type mixer Purified water was then added and granulation was carried out; drying and size adjustment then gave excipient granules.
201.6 g of the aforementioned adsorbed granules, 990 g of the excipient granules, 1.3 g of Menthol Coaton, 2.7 g of stevia, and 3.6 g of magnesium stearate were thoroughly mixed and then tabletted at a compression force of 1000 kg using a rotary tabletting machine (AP-15, Hata Ironworks Co., Ltd.) to give tablets (684 mg per tablet).

### Test Example 1

The disintegration time was measured on the tablets of Examples 1 to 6 and Comparative Examples 1 to 4 using the disintegration test procedure described in the Japanese Pharmacopoeia, Fourteenth Edition. The hardness of each tablet was also measured using a Kiya hardness meter (KHT-20N, Fujiwara Scientific Co., Ltd.). The results are shown in Tables 1 and 2.

**Table 1**

| | Example 1 | Example 2 | Example 3 | Example 4 | Example 5 | Example 6 |
|---|---|---|---|---|---|---|
| weight (mg) | 695 | 566 | 566 | 576 | 600 | 600 |
| compression force (kg) | 1000 | 650 | 550 | 380 | 600 | 600 |
| hardness (N) | 43 | 57 | 50 | 35 | 37 | 45 |
| disintegration time (sec.) | 20 | 31 | 15 | 17 | 17 | 16 |

**Table 2**

| | Comparative Example 1 | Comparative Example 2 | Comparative Example 3 | Comparative Example 4 |
|---|---|---|---|---|
| weight (mg) | 700 | 700 | 300 | 684 |
| compression force (kg) | 330 | 800 | 35 | 1000 |
| hardness (N) | 35 | 40 | 15 | 21 |
| disintegration time (sec.) | 77 | 14 | 50 | 37 |

The above results demonstrate that the orally rapid disintegrating tablet according to the present invention is an excellent formulation that has a satisfactory hardness and a very short disintegration time. The tablet of Comparative Example 2, while having an excellent hardness and disintegration time, exhibited bleed out of the active ingredient, as described below.

### Test Example 2

The tablets of Examples 1 to 4 and Comparative Examples 1 to 4 were held for 1 week at 60°C / 75% RH, the presence / absence of bleed out of the active ingredient onto the tablet surface was then visually inspected. The results are shown in Table 3.

**Table 3**

| | Example 1 | Example 2 | Example 3 | Example 4 |
|---|---|---|---|---|
| bleed out | no | no | no | no |
| | Comparative Example 1 | Comparative Example 2 | Comparative Example 3 | Comparative Example 4 |
| bleed out | yes | yes | yes | yes |

### Example 7

4 kg of calcium silicate was placed into a stirred-type mixer (100-L Super Mixer, Kawata Mfg. Co., Ltd.); 4.08 kg of teprenone and 8 g of tocopherol were added thereto and adsorption was carried out; and a solution of 0.8 kg of hydroxypropyl cellulose (HPC-L) dissolved in purified water was thereafter added and granulation was carried out. The product was dried with a fluid bed dryer (Flow Coater FLO-15, Freund Corporation) followed by size adjustment with a Power Mill to give adsorbed granules.
444.4 g of these adsorbed granules, 100 g of hydrated silicon dioxide (Sylysia, Fuji Silysia Chemical Co., Ltd.), 100 g of low-substituted hydroxypropyl cellulose (L-HPC, Shin-Etsu Chemical Co., Ltd.), 600 g of mannitol (Pearlitol DC500, Roquette Japan), 920 g of crystalline cellulose (Avicel PH102, Asahi Kasei Chemicals Corporation), 160 g of corn starch, 60 g of aspartame (Ajinomoto), 1.2 g of iron oxide yellow (Junsei Chemical Co., Ltd.), and 12 g of sodium stearyl fumarate were mixed and tabletted using a rotary tabletting machine at a compression force of 900 kg to give tablets (600 mg per tablet).

### Example 8

47.25 kg of hydrated silicon dioxide was placed in a stirred-type mixer (1000-L Super Mixer, Kawata Mfg. Co., Ltd.) and a solution of 9.45 kg of hydroxypropyl cellulose dissolved in an appropriate amount of purified water was added and granulation was carried out. Granules were obtained by drying with a fluid bed dryer (Flow Coater FLO-120, Freund Corporation) followed by size adjustment with a size adjustment apparatus (Speed Mill 300, Showa Giken Ltd.). 45 kg of teprenone and 0.09 kg of tocopherol were adsorbed onto these granules using a fluid bed dryer to give adsorbed granules. A mixture of these adsorbed granules with 11.25 kg of hydrated silicon dioxide (Sylysia), 103.95 kg of crystalline cellulose (Ceolus 802, Asahi Kasei Chemicals Corporation), 67.5 kg of mannitol (Pearlitol DC500), 18.00 kg of corn starch, 11.25 kg of low-substituted hydroxypropyl cellulose, 6.75 kg of aspartame, and 1.35 kg of sodium stearyl fumarate was tabletted using a rotary tableting machine at a compression force of 2100 kg to give tablets (607 mg per tablet).

### Comparative Example 5

Tablets (600 mg per tablet) were obtained as in Example 7, but in this case changing low-substituted hydroxypropyl cellulose to crosspovidone (Polyplasdone XL, ISP Japan), changing the 600 g of mannitol (Pearlitol DC500) to 1080 g of Mannitol S (Towa Kasei), and changing 920 g of crystalline cellulose to 440 g.

### Comparative Example 6

Tablets (600 mg per tablet) were obtained as in Example 7, but in this case omitting the corn starch and low-substituted hydroxypropyl cellulose, changing mannitol from 600 g to 1060 g, and changing crystalline cellulose from 920 g to 720 g.

### Test Example 3

The tablets in Examples 7 and 8 and Comparative Examples 5 and 6 were subjected to measurement of the hardness and disintegration time by the same procedures as in Test Example 1. The results are shown in Table 4.

**Table 4**

| | Example 7 | Example 8 | Comparative Example 5 | Comparative Example 6 |
|---|---|---|---|---|
| weight (mg) | 600 | 607 | 600 | 600 |
| compression force (kg) | 900 | 2100 | 900 | 900 |
| hardness (N) | 53 | 52 | 18 | 42 |
| disintegration time (sec.) | 27 | 18 | 8 | 44 |

As shown above, the tablet of Comparative Example 5, while having a short disintegration time, was unable to develop adequate hardness and was thus susceptible to abrasion. The tablet of Comparative Example 6 had a longer disintegration time than the tablets according to Examples 7 and 8, and also had a lower hardness.

The tablet obtained in accordance with the present invention by making a granule in which the fat-soluble active ingredient is adsorbed on an adsorbent and tableting a mixture that combines this granule, a saccharide, crystalline cellulose, and a lubricant, provides the orally rapid disintegrating tablet that exhibits a short disintegration time and a satisfactory strength and that is free of bleed out of the active ingredient.

## Claims

1. An orally rapid disintegrating tablet preparation obtained by tabletting a mixture comprising a saccharide, crystalline cellulose, a lubricant, and a granule of a fat-soluble active ingredient adsorbed to an adsorbent.

2. The orally rapid disintegrating tablet preparation according to Claim 1, wherein the fat-soluble active ingredient is at least one selected from the group consisting of vitamin A, vitamin D, vitamin E, vitamin K, teprenone, and coenzyme Q.

3. The orally rapid disintegrating tablet preparation according to Claim 1, wherein the fat-soluble active ingredient is an oily active ingredient.

4. The orally rapid disintegrating tablet preparation according to Claim 3, wherein the oily active ingredient is vitamin A, vitamin E, or teprenone.

5. The orally rapid disintegrating tablet preparation according to any of Claims 1 to 4, wherein the fat-soluble active ingredient is contained at from 5 to 75 % by weight based on a total weight of the tablet preparation.

6. The orally rapid disintegrating tablet preparation according to any of Claims 1 to 5, wherein the adsorbent is at least one selected from the group consisting of calcium silicate, hydrated silicon dioxide, and light anhydrous silicic acid.

7. The orally rapid disintegrating tablet preparation according to any of Claims 1 to 6, wherein the saccharide is a saccharide alcohol.

8. The orally rapid disintegrating tablet preparation according to Claim 7, wherein the saccharide alcohol is erythritol or mannitol.

9. The orally rapid disintegrating tablet preparation according to Claim 7, wherein the saccharide alcohol is mannitol.

10. The orally rapid disintegrating tablet preparation according to any of Claims 7 to 9, wherein an average particle diameter of the saccharide alcohol is from 300 µm to 700 µm.

11. The orally rapid disintegrating tablet preparation according to any of Claims 7 to 10, wherein a content of the saccharide alcohol is from 5 to 50 % by weight based on of a total weight of the tablet preparation.

12. The orally rapid disintegrating tablet preparation according to any of Claims 7 to 11, wherein the crystalline cellulose is contained at from 1 to 20 weight parts based on 1 weight part of the saccharide alcohol.

13. The orally rapid disintegrating tablet preparation according to any of Claims 1 to 12, wherein the adsorbent is contained at from 0.1 to 10 weight parts based on 1 weight part of the fat-soluble active ingredient.

14. The orally rapid disintegrating tablet preparation according to any of Claims 1 to 12, wherein the adsorbent is contained at from 0.5 to 2 weight parts based on 1 weight part of the fat-soluble active ingredient.

15. The orally rapid disintegrating tablet preparation according to any of Claims 1 to 14, wherein a hardness of the table preparation is at least 30 N and an intraoral disintegration time in the disintegration test procedure described in the Japanese Pharmacopoeia, Fourteenth Edition, is not more than 30 seconds.

16. A method of stabilizing an orally rapid disintegrating tablet preparation, comprising the steps of:
adding a saccharide, crystalline cellulose, and a lubricant to and mixing same with a granule comprising a fat-soluble active ingredient adsorbed on an adsorbent; and
tabletting the mixture.

17. A process of producing an orally rapid disintegrating tablet preparation, comprising the steps of:
adding a saccharide, crystalline cellulose, and a lubricant to and mixing same with a granule comprising a fat-soluble active ingredient adsorbed on an adsorbent; and
tabletting the mixture.
